(19) **Europäisches Patentamt**

**European Patent Office**

**Office européen des brevets**

(11) **EP 0 984 005 A1**

(12) **EUROPÄISCHE PATENTANMELDUNG**

(43) Veröffentlichungstag:
08.03.2000 Patentblatt 2000/10

(51) Int. Cl.[7]: **C07D 213/79**, C07D 213/80,
C07B 41/08

(21) Anmeldenummer: **99116538.2**

(22) Anmeldetag: **24.08.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **01.09.1998 DE 19839559**

(71) Anmelder:
**Degussa-Hüls Aktiengesellschaft**
**60311 Frankfurt am Main (DE)**

(72) Erfinder:
- **Heinz, Dieter, Dr.**
  **51375 Leverkusen (DE)**
- **Hölderich, Wolfgang, Prof. Dr.**
  **67227 Frankenthal (DE)**
- **Krill, Steffen, Dr.**
  **67346 Speyer (DE)**
- **Böck, Wolfgang, Dr.**
  **63505 Langenselbold (DE)**
- **Huthmacher, Klaus, Dr.**
  **63571 Gelnhausen (DE)**

(54) **Verfahren zur Herstellung von Nikotinsäure**

(57) Die Erfindung betrifft ein verbessertes Verfahren zur Herstellung von Nikotinsäure durch Direktoxidation von β-Picolin in der Gasphase, wobei Wasser und β-Picolin getrennt voneinander zum Katalysatorbett geführt werden und der Katalysator auf einem Titandioxidträger basiert, der nach der Sulfatmethode hergestellt wurde und eine hohe spezifische Oberfläche und einen Anteil von 5 - 50 % Vanadiumoxid besitzt.

EP 0 984 005 A1

**Beschreibung**

Die vorliegende Erfindung betrifft ein neues und verbessertes Verfahren zur Herstellung von Nikotinsäure durch Direktoxidation von β-Picolin in der Gasphase, wobei Wasser und β-Picolin getrennt voneinander zum Katalysatorbett geführt werden und der Katalysator auf einem Titandioxidträger, der nach der Sulfatmethode hergestellt ist und eine hohe spezifische Oberfläche besitzt und einem Anteil von 5 - 50 % Vanadiumoxid basiert.

Nikotinsäure ist weit verbreitet in den Anwendungsbereichen Medizin und Landwirtschaft, als Vitamin sowie als Zwischenprodukt für Pharmaka und Wachstumsregulatoren von Pflanzen.

Für die Synthese von Nikotinsäure aus dem β-Picolin sind verschiedene Verfahren bekannt. Zum einen die Flüssigphasen-Direktoxidation an $HNO_3$ und $H_2SO_4$ (USP 2586555, 1952) bei Temperaturen von 75 - 300 °C und Ausbeuten von 66 - 77 %. Nachteile dieser Verfahren sind die hohe anfallende Salzfracht sowie die Erzeugung großer Abwasserströme. Ein mikrobiologisches Verfahren zur Oxidation des β-Picolin zur Nikotinsäure (EP 442430, 1995) erreicht nach 16 h Reaktionszeit eine Ausbeute von 50 %, wobei die unbefriedigende Raum-Ziet Ausbeute und die aufwendige Abtrennung der Biomasse von der Nikotinsäure eine industrielle Anwendung unvorteilhaft erscheinen lassen.

In der Gasphase kennt man die Ammonoxidation des β-Picolins zum 3-Cyanopyridin mit anschließender Hydrolyse zur Nikotinsäure (USSR Inventor's Certificate No. 235764, 1969). Nachteil dieses Verfahrens ist die Notwendigkeit zweier Prozeßstufen sowie eine zusätzliche Abtrennung der Nikotinsäure vom Produktgemisch mittels Kristallisation. Als Gesamtausbeute an Nikotinsäure werden 86 - 88 % genannt. Ebenfalls in der Gasphase gibt es einige Untersuchungen zur Direktoxidation des β-Picolins an Vanadiumoxidkatalysatoren. Die besten Ergebnisse werden mit 82 - 86 % Ausbeute an Nikotinsäure bei Zugabe von Luft und Wasser und Temperaturen von 250 - 290 °C genannt (EP 747 359, WO 95/20577, 1995). Die Vorteile der letzten Verfahrensvariante sind der Verzicht auf Hilfsstoffe oder Lösungsmittel mit Ausnahme der unkritischen Substanzen Wasser und Luft zur Zugabe von Sauerstoff. Jedoch liegen die Ausbeuten an Nikotinsäure immer noch deutlich unter 90 %. Damit ist dieses Verfahren noch nicht wirtschaftlich genug.

Der Erfindung lag daher die Aufgabe zugrunde, diese Direktoxidation des β-Picolins zur Nikotinsäure weiter zu verbessern und Ausbeuten von ≥ 90 % zu erzielen, um die Wirtschaftlichkeit des Verfahrens deutlich zu verbessern.

Demgemäß wurde überraschend dadurch ein neues und verbessertes Verfahren zur Herstellung von Nikotinsäure durch Direktoxidation von β-Picolin gefunden, daß man β-Picolin getrennt vom Wasser zum Reaktor zuführt und erst am Beginn des Katalysatorbettes zusammenführt, und einen vanadiumoxidhaltigen Katalysator einsetzt, dessen Träger aus einem nach der Sulfatmethode hergestellten Titandioxid mit hoher Oberfläche (> 100 $m^2$/g) besteht, wobei der Vanadiumoxidanteil zwischen 5 und 50 wt.-% beträgt. Als Sauerstoffquelle wird Luft verwendet, es kann aber auch reiner Sauerstoff oder eine variierende Mischung aus Sauerstoff und Stickstoff verwendet werden. Die Luft wird entweder zusammen mit dem β-Picolin, mit dem Wasser oder ebenfalls getrennt zum Katalysator geführt. Besonders bevorzugt ist die Zugabe von $CO_2$ zum Eduktfeed, wodurch die Selektivität der Reaktion weiter gesteigert werden kann.

Die Ausbeuten sind besonders hoch (bis zu 95 %), wenn die spezifische Oberfläche des Titandioxidträgers über 100 $m^2$/g, vorzugsweise über 250 $m^2$/g beträgt, wenn der Katalysator einen Sulfatgehalt größer 0,1 % besitzt und der Gehalt an Vanadiumoxid zwischen 5 und 50 %, vorzugsweise zwischen 10 und 30 % liegt. Das Titandioxid liegt vorteilhaft hauptsächlich in der Anatas-Form vor.

Bei der industriellen Herstellung von Titandioxiden unterscheidet man zwei Verfahren, das Chlorid- und das Sulfatverfahren (Ullmann's Encyklopädie der technischen Chemie, 4. Auflage, VCH Weinheim, Band 18, S. 569). Das Chloridverfahren besteht aus den Schritten Chlorierung, Kühlung, $TiCl_4$-Reinigung, $TiCl_4$-Verbrennung und $TiO_2$-Abscheidung. Das Sulfatverfahren besteht in der Auflösung des Titanrohstoffes in konzentrierter Schwefelsäure und anschließender Ausfällung des Titandioxids. Im einzelnen besteht dieses Verfahren aus den Schritten Aufschluß, Lösen und Reduktion, Klärung, Kristallisation und Hydrolyse. Beiden Verfahren schließen sich noch Aufbereitungsverfahren zur Gewinnung eines möglichst reinen Produktes an. Charakteristisches Merkmal des Sulfatverfahrens ist vor allem der geringe Sulfatrestgehalt, der im Titandioxid enthalten ist, und durch Auswaschen zwar reduziert aber nicht vollkommen vermieden werden kann. Verbunden damit sind auch unterschiedliche chemische Eigenschaften (G.A. Zenkovets, A.M. Volodin, A.F. Bedilo, E.F. Burgina, E.M. Al'kaeva, Kinetics and Catalysis, Vol. 38 (1997) pp.669).

Die erfindungsgemäße Direktoxidation des β-Picolin zur Nikotinsäure wird bei einer Reaktionstemperatur zwischen 150 und 450 °C, vorzugsweise bei 200 und 325 °C und ganz besonders bei 240 bis 290 °C in einem Festbettreaktor durchgeführt, alternativ sind auch die Fahrweise in einem Wirbelbett, Moving-bed sowie in der Flüssigphase im Festbett und mehrphasig im Trickleben bzw. Autoklaven möglich. Das molare Wasser/ β-Picolin-Verhältnis sollte zur Erzielung guter Ergebnisse zwischen 15 und 100, insbesondere zwischen 25 und 75 liegen, das molare Sauerstoff/β-Picolin-Verhältnis zwischen 5 und 40, insbesondere zwischen 10 und 35, sowie die Katalysatorbelastung bei einer WHSV

(Weight hourly space

$$\text{velocity} = \frac{m_{Edukr}/h}{m_{Katalysator}} \quad [h^{-1}])$$

von 0,02 bis 5 $h^{-1}$, insbesondere zwischen 0,04 und 1 $h^{-1}$ und ganz besonders zwischen 0,05 und 0,5 $h^{-1}$.

Aufgrund der hohen Ausbeuten und der leichten Abtrennbarkeit der Nikotinsäure vom restlichen Produktgemisch (Sublimationstemperatur der Nikotinsäure = 235 °C) kann das synthetisierte Produkt bei 100 - 230 °C in hoher Reinheit abgetrennt werden, das Wasser, die Gasanteile (vornehmlich Sauerstoff und Stickstoff), das Zwischenprodukt Pyridin-3-carbaldehyd sowie nicht umgesetztes β-Picolin können gasförmig recycliert werden und zu Beginn des Katalysatorbettes mit frischem β-Picolin und Sauerstoff zusammengeführt werden. Das als Hauptnebenprodukt durch Totaloxidation entstehende $CO_2$ kann dabei mit zurückgeführt werden, da wir feststellen konnten, daß die Zugabe eines zusätzlichen Feedgasstromes bzw. im Regasstromes, der z.B. $N_2$, Ar, CO, $CH_4$, $N_2O$ und ganz besonders $CO_2$ enthält, die Ausbeute der Reaktion, die bei höheren Belastungen absinkt, deutlich steigern kann.

Als Trägermaterialien werden verschiedene kommerziell erhältliche Titandioxide eingesetzt, die zum einen nach der Chlorid-Methode als auch nach dem Sulfatverfahren hergestellt wurden. Auch nach anderen Fällungsverfahren hergestellte Titandioxide können verwendet werden. Sie unterscheiden sich in ihrer spezifischen Oberfläche, die durch $N_2$-Sorption und BET-Auswertung bestimmt wurde, sowie in ihrem Sulfatgehalt.

Diese Trägermaterialien wurden direkt mit Vanadiumoxid beladen nach der folgenden Methode:

Als Vanadiumprecursor wird eine wasserlösliche Vanadiumverbindung gewählt, allgemein werden Ammonium-metavanadat, Vanadium-acetylacetonat oder Vanadiumoxalat verwendet. Entsprechend der gewünschten $V_2O_5$-Beladung wird die dazu benötigte Menge des Vanadiumprecursors in eine wäßrige Lösung überführt. Mit dieser wäßrigen Lösung wird das Titandioxid-Trägermaterial gegeben und verrührt. Nach einem Verdampfungsschritt, in dem das Wasser abgetrennt wird, liegt bei hinreichender Durchmischung ein Feststoff vor, der aus dem Titandioxid und dem darauf adsorbierten Vanadiumprecursor besteht. Das pulverförmige Produkt kann anschließend durch Temperung konditioniert werden,womit der gewünschte Katalysator $V_2O_5$/$TiO_2$ vorliegt.

Das erfindungsgemäße Verfahren wird durch die folgenden Beispiele belegt.

Als Trägermaterialien wurden die folgenden Titandioxide eingesetzt:

Tabelle 1

| Trägermaterialien | | | |
|---|---|---|---|
| Titandioxid (Hersteller) | BET-Oberfläche ($m^2$/g) | Haupt-Kristallformati on | Sulfatgehalt |
| $TiO_2$-P25 (Degussa) | 50 | Anatas | 0 |
| Hombitec K01 (Sachtleben Chemie) | 129 | Anatas | 0,5 % |
| Hombitec K03 (Sachtleben Chemie) | 130 | Anatas | 1,5 % |
| Hombifine N (Sachtleben Chemie) | 275 | Anatas | 0,5 % |
| Hombifine N* (Sachtleben Chemie) | 260 | Anatas | 1,5 % |

Aus den Trägermaterialien nach Tabelle 1 wurden nach der im folgenden beschriebenen Imprägniertechnik die in Tabelle 2 aufgeführten $V_2O_5$-$TiO_2$-Katalysatoren hergestellt.

Als Vanadiumprecursor wurde einheitlich Ammonium-metavanadat verwendet. Entsprechend der angegebenen Soll-Beladung wurde die dazu benötigte Menge Ammonium-metavanadat in destilliertem Wasser aufgelöst. Zur besseren Löslichkeit des Feststoffes wurde das Wasser auf ca. 40 °C erhitzt. Zu dieser wäßrigen Lösung wird die gewünschte Menge des Titandioxids gegeben und verrührt. In einem Rotationsverdampfer wird unter ständigem Rühren und Wasserstrahlpumpen-Vakuum bei einer Ölbad-Temperatur von 100 °C das Wasser abrotiert. Das pulverförmige Produkt wird danach bei 450 °C 4 h lang kalziniert und zu Tabletten von ca. 13 mm Durchmesser verpresst. Aus diesen Tabletten wird dann in einer Zerkleinerungsprozedur die Split-Fraktion von 1 - 1,6 mm herausgesiebt, welche als Katalysator eingesetzt wird.

| Katalysator | Träger | BET-Oberfläche ($m^2$/g) | $V_2O_5$-Gehalt (soll) | $V_2O_5$-Gehalt (ist) |
|:---:|:---:|:---:|:---:|:---:|
| A | $TiO_2$-P25 | 25 | 2,5 | 2,3 |
| B | Hombitec KO1 | 65 | 18 | 17 |
| C | Hombitec K03 | 61 | 18 | 12 |
| D | Hombifine N | 40 | 20 | 19 |
| E | Hombifine N* | 35 | 20 | 20 |

Beispiele:

Beispiel 1 (Vergleichsbeispiel):

Als Reaktor wird ein Rohrwendelreaktor aus Stahl verwendet, der in einem Umluftofen auf Reaktionstemperatur gehalten wird. Es werden 3 g Katalysator A eingefüllt. Als Sauerstoffquelle wird Luft verwendet. Wasser und β-Picolin werden als Gemisch eingesetzt und gemeinsam zum Reaktor gepumpt, dort mit der Luft verdampft und zum Katalysator geführt. Bei einer Reaktionstemperatur von 265 °C, einer WHSV von 0,06 g/h β-Picolin / g Katalysator (=0,06 $h^{-1}$) und einem molaren Verhältnis $O_2$/$H_2O$/β-Picolin = 40/100/1 wurde ein β-Picolin-Umsatz von 61,6 % gemessen bei einer Selektivität zur Nikotinsäure von 22 % (molar).

Beispiel 2 (Vergleichsbeispiel):

Die gleiche Versuchsanordnung wie in Beispiel 1 wurde ohne Katalysator wiederholt. Bei einer Temperatur von 275 °C, einem $O_2$/$H_2O$/β-Picolin - Verhältnis von 40/70/1 (molar) und einer Fördermenge ($H_2O$ + β-Picolin) = 4,4 g/h wurde ein β-Picolin-Umsatz von 30 % zu gasförmigen Zersetzungsprodukten gemessen.

Beispiel 3 (Vergleichsbeispiel):

Der Versuch von Beispiel 2 wurde wiederholt, jedoch wurde diesmal kein Wasser zugegeben. Bei einer Temperatur von 275 °C, einem $O_2$/β-Picolin-Verhältnis von 40/1 und einem Massenstrom von β-Picolin = 0,325 g/h wurden nur noch 10 % β-Picolin-Verlust gemessen.

Beispiel 4 (erfindungsgemäß):

Der im Beispiel 1 beschriebene Aufbau der Versuchsanlage wurde dahingehend geändert, daß die Luft mit β-Picolin in einem temperierten Sättiger angereichert wurde, während das Wasser separat erhitzt und direkt zum Katalysatorbett gefördert und erst dort mit dem Luft / β-Picolin - Gemisch zusammengeführt wird. Eingesetzt wurde wieder Katalysator A (8,91 g). Bei einer Reaktionstemperatur von 265 °C einer WHSV von 0,05 $h^{-1}$ und einem $O_2$/$H_2O$/ β-Picolin - Verhältnis von 40/70/1 wurde ein Umsatz von 59,9 % und eine Selektivität zur Nikotinsäure von 53,4 % erzielt. Die Verbesserung zum Ergebnis des Beispiels 1 bestätigt die in den Vergleichsbeispielen beobachtete Zersetzung des β-Picolin in Anwesenheit des Wassers - unabhängig vom Katalysator.

Beispiele 5-14

In den weiteren erfindungsgemäßen Beispielen wird daher immer das Luft / β-Picolin - Gemisch getrennt vom Wasser zum Beginn des Katalysatorbettes geführt. Beispiel 13 zeigt, daß geringere Wasser- und Luftüberschüsse sowie eine hohe Beladung (WHSV) zu geringeren Ausbeuten führen, die durch Zugabe von $CO_2$ verbessert werden können (Beispiel 14).

| | | | | | | | | | |
|---|---|---|---|---|---|---|---|---|---|
| Beispiele 5-14: | | | | | | | | | |
| Nr. | Kataly -sator | T [°C] | $O_2/H_2O/\beta$-Picolin (molar) | $CO_2/\beta$-Picolin (molar) | WHSV [$h^{-1}$] | $m_{Katalysator}$ [g] | Umsatz [%] | Selekivität [mol-%] | |
| 5 | D | 275 | 35/60/1 | - | 0,04 | 6,5 | 97 | 98 | |
| 6 | D | 265 | 35/70/1 | - | 0,04 | 6,5 | 98 | 98 | |
| 7 | D | 290 | 35/60/1 | - | 0,045 | 6,5 | 100 | 49 | |
| 8 | E | 265 | 40/70/1 | - | 0,04 | 6,5 | 96 | 96 | |
| 9 | B | 265 | 35/60/1 | - | 0,046 | 6,1 | 100 | 84 | |
| 10 | C | 265 | 35/55/1 | - | 0,044 | 7,1 | 99 | 75 | |
| 11 | D | 275 | 30/60/1 | - | 0,025 | 6,5 | 100 | 62 | |
| 12 | D | 275 | 22/56/1 | - | 0,085 | 6,5 | 97 | 75 | |
| 13 | D | 275 | 22/50/1 | - | 0,11 | 3,9 | 96 | 63 | |
| 14 | D | 275 | 22/50/1 | 9/1 | 0,11 | | 93 | 72 | |

**Patentansprüche**

1. Verfahren zur Herstellung von Nikotinsäure durch Direktoxidation aus β-Picolin,
**dadurch gekennzeichnet,**

   daß β-Picolin und Wasser getrennt dem Katalysatorbett, gefüllt mit einem vanadiumhaltigen $TiO_2$-Trägerkatalysator, zugeführt werden.

2. Verfahren nach Anspruch 1,
**dadurch gekennzeichnet,**
daß der Heterogenkatalysator als Trägermaterial Anatas mit einer spezifischen Oberfläche von mehr als 100 $m^2$/g enthält.

3. Verfahren nach Anspruch 1,
**dadurch gekennzeichnet,**
daß der Anatas-Träger nach der Sulfatmethode hergestellt ist.

4. Verfahren nach Anspruch 1,
**dadurch gekennzeichnet,**
daß Titandioxid-Träger einen Sulfatgehalt von mehr als 0,1 % besitzt.

5. Verfahren nach Anspruch 1,
**dadurch gekennzeichnet,**
daß der Vanadiumoxidgehalt zwischen 5 und 50 % beträgt.

6. Verfahren nach Anspruch 1,
**dadurch gekennzeichnet,**
daß die Reaktionstemperatur zwischen 150 und 450 °C liegt.

7. Verfahren nach Anspruch 1,
**dadurch gekennzeichnet,**
daß das Sauerstoff/Edukt Verhältnis zwischen 5/1 und 40/1, insbesondere zwischen 10/1 und 35/1 und das Wasser/Edukt Verhältnis zwischen 15/1 und 100/1, insbesondere zwischen 25/1 und 75/1 liegt.

8. Verfahren nach Anspruch 1,

**dadurch gekennzeichnet,**
daß die WHSV (weight hourly space velocity) zwischen 0,02 und 5 h$^{-1}$ liegt.

9. Verfahren nach Anspruch 1,
**dadurch gekennzeichnet,**
daß die Reaktion in der Gasphase oder Flüssigphase in einem Festbettreaktor, Wirbelbettreaktor oder Movingbed-Reaktor stattfindet oder mehrphasig in einem Tricklebedreaktor oder Autoklav durchgeführt wird.

10. Verfahren nach Anspruch 1,
**dadurch gekennzeichnet,**
daß die Zugabe von $CO_2$ zur Reaktionsmischung eine zusätzliche Verbesserung der Nikotinsäureausbeute bewirkt.

11. Verfahren nach Anspruch 1,
**dadurch gekennzeichnet,**
daß bei den hohen Ausbeuten von ≥ 90 % durch die leichte Abtrennbarkeit der Nikotinsäure, die bei ca. 235 °C desublimiert, das restliche Prozeßgas zurückgeführt werden kann und mit frischem Edukt und Sauerstoff wieder dem Katalysatorbett zugeführt werden kann.

**Europäisches Patentamt**

## EUROPÄISCHER RECHERCHENBERICHT

**Nummer der Anmeldung**

EP 99 11 6538

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| A,D | EP 0 747 359 A (BORESKOVA INST KATALIZA SIBIR) 11. Dezember 1996 (1996-12-11) * Anspruch 1; Beispiele * --- | 1 | C07D213/79 C07D213/80 C07B41/08 |
| A | JAERAES S ET AL: "PREPARATION OF PYRIDINEMONOCARBOXYLIC ACIDS BY CATALYTIC VAPOUR PHASE OXIDATION OF ALKYLPYRIDINES. I. OXIDATION OF 2- AND 3-PICOLINE AND DECARBOXYLATION OF 2- AND 3-PYRIDINECARBOXYLIC ACID IN THE VAPOUR PHASE" JOURNAL OF APPLIED CHEMISTRY AND BIOTECHNOLOGY,GB,BLACKWELL SCIENTIFIC PUBLICATIONS LTD. OXFORD, Bd. 27, Nr. 10, Seite 499-509 XP000644583 * Seite 507 * --- | 1 | |
| A | CH 543 510 A (TEIJIN CHEMICALS LTD.) 14. Dezember 1973 (1973-12-14) * Beispiel 6 * ----- | 1 | |

| | RECHERCHIERTE SACHGEBIETE (Int.Cl.7) |
|---|---|
| | C07D C07B |

Der vorliegende Recherchenbericht wurde für alle Patentansprüche erstellt

| Recherchenort | Abschlußdatum der Recherche | Prüfer |
|---|---|---|
| DEN HAAG | 21. Dezember 1999 | Bosma, P |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie,übereinstimmendes Dokument

EPO FORM 1503 03 82 (P04C03)

**ANHANG ZUM EUROPÄISCHEN RECHERCHENBERICHT
ÜBER DIE EUROPÄISCHE PATENTANMELDUNG NR.**  EP 99 11 6538

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

21-12-1999

| Im Recherchenbericht angeführtes Patentdokument | Datum der Veröffentlichung | Mitglied(er) der Patentfamilie | Datum der Veröffentlichung |
|---|---|---|---|
| EP 0747359 A | 11-12-1996 | RU 2049089 C | 27-11-1995 |
| | | JP 9508379 T | 26-08-1997 |
| | | US 5728837 A | 17-03-1998 |
| | | CN 1139922 A | 08-01-1997 |
| | | WO 9520577 A | 03-08-1995 |
| CH 543510 A | 14-12-1973 | KEINE | |

EPO FORM P0461

Für nähere Einzelheiten zu diesem Anhang : siehe Amtsblatt des Europäischen Patentamts, Nr.12/82